# EUROPEAN PATENT APPLICATION

(11) **EP 0 954 984 A1**
(43) Date of publication of application: **10.11.1999**
(21) Application number: 98850068.2
(22) Date of filing: 04.05.1998
(51) Int. Cl.: A23L 1/308, A61K 35/78

(54) **Dietary fibre composition**

(71) Applicant: Källna Specialprodukter AB, 205 03 Malmö (SE); Alabaster, Oliver, Alexandria, VA 22309 (US)
(72) Inventor: Alabaster, Oliver, Alexandria, VA 22309 (US)
(74) Representative: Wiklund, Ingrid Helena

(57) **Abstract**

A dietary fibre composition comprising any two or three components or derivative subfractions of wheat bran, soy and flaxseed is described. A synergistic effect is shown between any two or three of the above components in inhibiting colon cancer in a rat model in comparison with the effect of the components alone.

## Description

The present invention relates to a dietary fibre composition comprising as main ingredients any two or three components of wheat bran, soy and flaxseed.

During human evolution fibre intake was much higher, and fat intake was much lower than it is today. In fact, the modern western diet has a dramatically different dietary balance than the diet our bodies were designed for. Until about 150 years ago our traditional diet was much closer to what it should be. Since 80-90 % of our food traditionally came from plants, heart diseases and cancer were very rare. Now, in affluent societies when this number is closer to 50-60%, heart disease, cancer and a host of other diseases are much more common.

Colon cancer is one of the most common forms of cancer in the western world. Substantial evidence suggests that diet is a significant or even the main factor responsible for the high incidence and mortality due to colon cancer in western countries (Diet, Nutrition and Cancer. (1982). National Academy of Sciences, Washington DC; Doll, R. and Peto, R. (1981) . The causes of cancer: Quantitative assessment of avoidable risk of cancer in the United States today. J. Natl. Cancer Inst., 66, 1191-1308) . A high fat diet is believed to be an important etiological factor in the causation of colon cancer (Doll, R et al, *ibid*.).

Further, epidemiological studies have shown that the intake of cereals or non starch polysaccharide fibre reduces the risk of developing colon cancer.

Even though it is widely known that dietary fat plays an important role in the etiology of colon cancer, the consumption of fatty foods in the western world has not significantly declined. Consequently, since the treatment of colon cancer has not resulted in a significant reduction in mortality over the last decades, a preventive approach to reducing the incidence of colon cancer that does not depend only upon reducing dietary fat intake is needed.

Dietary fibres have been extensively studied in laboratory animals for their colon cancer preventive potential. Most importantly, certain dietary fibres have also been shown to reduce the incidence of colon tumors in rats that also consumed high fat western style diets (Greenwald, P., Lanza, E. (1980). Role of dietary fiber in the prevention of cancer. In: Devita, V.T., Hellman, S. and Rosenberg, S.A. (eds.), Important Advances in Oncology. Lippincott, J.B., Philadelphia, pp 37-54; Tang, ZC., Shivapurkar, N., Frost, A. and O. Alabaster. (1996). The effect of dietary fat on the promotion of mammary and colon cancer in a dual-organ rat carcinogenesis model. Nutrition and Cancer., Vol. 25, No. 2, 151-159; Alabaster, O., Tang, ZC. and N. Shivapurkar. (1997). Wheat bran cereals inhibit the development of aberrant crypt foci and colon tumors. Food and Chemical Toxicology. May; 35(5):517-522).

Dietary fibres are classified as soluble and insoluble fibres. Insoluble fibres are more commonly known for their anticancer potential (Greenwald, P. et al, *ibid*.), while soluble fibres are commonly known for their ability to reduce blood cholesterol. However, psyllium, which contains about 78% soluble fibres, has also been shown to inhibit chemically induced colon cancer in rodents (O. Alabaster et al, Potential synergism between wheat bran and psyllium: enhanced inhibition of colon cancer, Cancer Letters 75 (1993) 53-58; Greenwald, P., et al, *ibid*.). The effect of soy on breast cancer development has also been studied (Barnes, S. (1997) . The chemopreventive properties of soy isoflavonoids in animal models of breast cancer. Breast Cancer Treatment Nov-Dec; 46(2-3); 169-179) as has the effects of flax on cancer as well as on blood vessel disease (Katadore, M., Osborne, MP., and N.T. Telang. (1998) Inhibition of aberrant proliferation and induction of apoptosis in pre-neoplastic human mammary epithelial cells by natural phytochemicals. Oncology Reports. MarApril;5(2):311-315; Prasad, K. Dietary flaxseed in prevention of hypercholesterolemic atherosclerosis. (1997) Atherosclerosis. Jul 11; 132(1):69-76).

A dietary fibre complement, called "bran cocktail", has been suggested (O. Alabaster, J. Jibrin, Bran Plan Diet, Rodale press 1993) which is said to help in losing weight, reducing digestive disorders, preventing heart disease, avoiding cancer and living longer. This fibre complement contained an uncooked mixture of wheat bran, oat bran, psyllium and rice bran in a weight ratio of 4:2:1:O.5.

Recently it has been shown (O. Alabaster et al, Potential synergism between wheat bran and psyllium: enhanced inhibition of colon cancer, Cancer Letters 75 (1993) 53-58) that there was an enhancement of the protective effect of wheat bran and psyllium against colon cancer in rats when the two types of fibres, that is unsoluble and soluble, respectively, were administered as a combination in the presence of a high fat western style diet. In this study, it was shown that a weight ratio in the range of 75:25 to 25:75 presented a 25% increase in effect compared to wheat bran or psyllium alone, while a weight ratio of 1:1 between wheat bran and psyllium fibre gave the optimal protection, that is a 50% increase in effect compared to the use of the same amount of wheat bran alone. Most important, the combination would still retain a remarkable blood cholesterol lowering ability. In a further study (L.A. Cohen, et al, Wheat Bran & Psyllium Diets: Effects on Mammary Tumorogenesis, JNCI (1996) July 3, 88 (13):899-907) there has also been shown a synergistic effect between wheat bran and psyllium in lowering estrogen and potentially inhibiting breast cancer in animals.

Psyllium is a cereal fibre grown in India, and therefore not readily obtainable, which makes it expensive. Furthermore, its taste is unappealing. Consequently there is a great need of a replacement for psyllium to be used in combination with wheat bran, which has the same beneficial effects together with being inexpensive and having a pleasant taste.

It has now unexpectedly been found that the same type of synergism can result from a combination of any two or three components of wheat bran, soy and flaxseed in certain weight ratios.

Thus, according to the invention, a dietary fibre composition is presented that comprises any two or three components or derivative subfractions of wheat bran, soy and flaxseed in an amount equivalent to a ratio of from 1:1 to 1:9 parts by weight of any or each component.

In further embodiments of the invention there is incorporated in the composition one or more further dietary fibre components chosen from the group comprising psyllium, rice bran and oat bran or derivative subfractions thereof in amounts equivalent to a ratio of from 0.1 to 4 parts per weight.

In order to further enhance the taste of the composition there can also be included small amounts of almond, e.g. in a ratio of from 0.1 to 4 parts per weight.

Due to the synergistic effect between any two or three of the main components in the composition, the amount of psyllium could be reduced or even excluded, thus saving costs and avoiding too much influence of the unpleasant taste of psyllium.

Beside the fibre components as such, also derivative subfractions of the fibre components having the same acitivity can be used.

In order to further broaden the effects, the composition could be enriched with vitamins, minerals, phytonutrients or other disease-inhibiting agents or nutrients.

In a preferred embodiment of the invention, the composition is cooked. This improves the taste of the composition, especially the flavour of psyllium when present. Cooking may also improve the storage quality of the product because it destroys any bacteria that might be in the uncooked product.

The individual ingredients in the composition according to the invention as well as embodiments thereof have the following actions/properties based on either experimental data and/or clinical data:
- Wheat bran:: Inhibits colon cancer. Inhibits colonic polyps. Lowers estrogen levels which could reduce breast cancer risk. Increases laxation. No effect on cholesterol. Insoluble fibre.
- Soy:: In the form of protein or flour. Contains cancer inhibitors.
- Flax seed:: Contains cancer inhibitors.
- Psyllium:: Lowers cholesterol. Reduces colon cancer risk. Increases laxation. Contains cancer inhibitors. 78% soluble fibre.
- Rice bran:: Lowers cholesterol.
- Oat bran:: Lowers cholesterol. No effect on colon cancer or laxation. The predominant fibre is soluble.

Two embodiments of the composition according to the invention have been tested in the animal colon cancer model, one comprising any combination of two or three of the fibre components wheat bran, soybean and flax seed in different proportions, the other comprising wheat bran, oat bran, psyllium, rice bran, soy flour and flax seed in certain proportions. The results of these experiments indicate that both combinations are as effective as the previously shown best synergistic combination of wheat bran and psyllium, even though they contained less than the synergistically optimal levels of wheat bran and psyllium or no psyllium at all.

The dietary fibre composition according to the invention can be used in a lot of ways as a daily food supplement. Thus, it can be added to yoghurt or to low fat milk shake. It can also easily be included in recipes for pancakes, waffles or bread. Another convenient way of adding the dietary fibre composition to one's food is to sprinkle it on salad, or to use it as a substitute for about 25% of the meat used for making hamburgers. It can also be added to soup or sprinkled on one's meal, such as pizza or pasta.

The invention will now be further illustrated in the following non limiting examples.

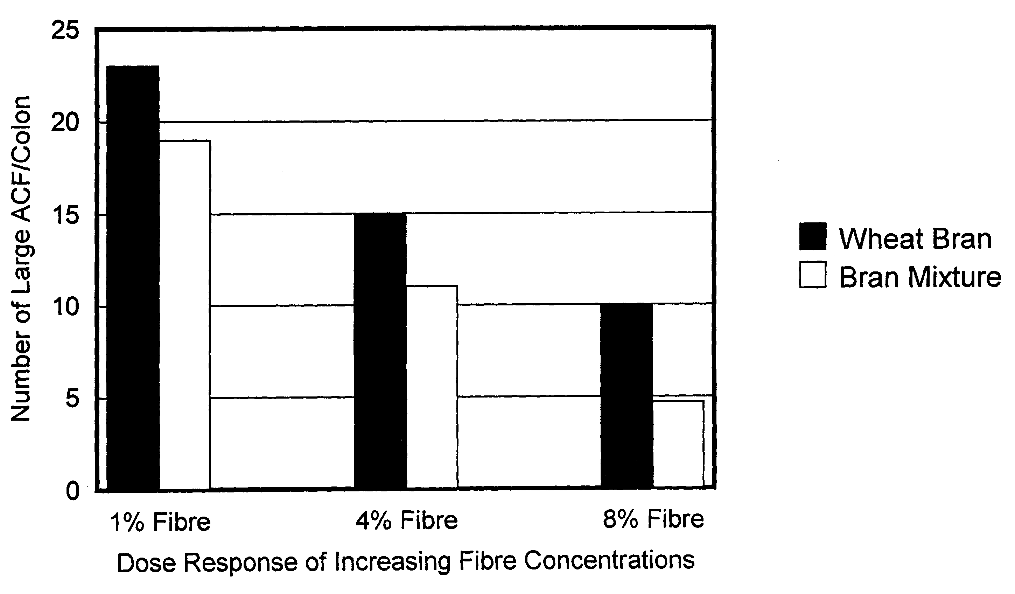

### BRIEF DESCRIPTION OF THE DRAWING

The figure is a graph showing the inhibitory effect on aberrant crypts (and colon cancer) of a bran mixture according to the invention in comparison with wheat bran as well as the dose response of increasing fibre concentration.

### ANIMAL COLON CANCER MODEL

In the following examples, use has been made of the following animal colon cancer model.

Colon cancer develops in a stepwise manner with each step resulting in the formation of a new altered cell population, until eventually one of those altered cell populations expresses the phenotypic characteristics of cancer. Early detection of cancer or the modulation of the carcinogenic process requires the detection of altered cell populations, or precancerous lesions that serve as intermediate biomarkers.

Conventional cancer prevention bioassays in rodents use tumors as the end point of cancer risk and therefore are time consuming and expensive. Consequently, specific precancerous lesions that have been consistently shown to predict cancer risk under different experimental conditions are potentially very valuable for use in rapid, inexpensive bioassays.

Distinct morphological lesions known as aberrant crypt foci (ACF) are specific biomarkers that can be used to predict colon cancer risk. ACF can be detected readily in unembedded segments of colons from rodents and humans at risk of developing colon cancer using methylene blue. These ACF are established as preneoplastic lesions in the development of colon cancer. In rodents and humans, ACF display mutations and histologic changes of colon tumors (Stopera, S.A., Carcinogenesis 13, 2081-2085, 1892; Pretlow, T.P. JNCI 85, 2004-2007, 1993; Losi, L., J. Pathol. 178, 259-263, 1996), and the growth of ACF correlates with the adenocarcinoma yield (Zhang, X.M., JNCI 84, 1026-1030, 1992; Pretlow, T.P., Carcinogenesis 13, 1509-1512, 1992; Magnuson, B.A., Cancer Res. 53, 4499-4504, 1993). The number of ACF per colon is an assay for initiators of colon cancers. A previous study (Shivapurkar, N., Tang, ZC., and O. Alabaster. (1992). The effect of high- and low-risk diets on aberrant crypt and colonic tumor formation in Fischer-344 rats. Carcinogenesis, 13, 887-890) and a subsequent study (Shivapurkar, N., Tang, ZC., Frost, A. and O. Alabaster. (1995). Inhibition of progression of aberrant crypt foci and colon tumor development by vitamin E and β-carotene in rats on a high-risk diet. Cancer Letters, 91, 125-132) have clearly demonstrated a direct correlation between the azoxymethane (AOM) induction of ACF and the subsequent development of colon tumors in rats fed high risk and low risk diets, or diets with and without a chemo-preventive agent. Subsequent studies have successfully employed ACF as intermediate biomarkers for colon cancer. Since individuals at higher risk for colon cancer are more likely to bear ACF in their colons before the intervention starts, the dietary agents that inhibit the progresson of ACF, or cause their reversal, are more likely to be effective in reducing the incidence of colon cancer. Thus, a bioassay based on ACF is expected to be very useful as a rapid and economical means to evaluate the colon cancer preventive potential of different combinations of dietary fibres under different dietary conditions.

In the following experiments ACF, which are clusters of abnormal cells that are the first step in the slow development of colon cancer, were measured. Since ACF are markers for colon cancer, a reduction in ACF is typically used by researchers to predict for a reduction in colon cancer development.

### EXAMPLE 1

The Fischer-344 male rat initiated with AOM has been one of the most commonly used in vivo models for studying colon cancer. In the experiments presented here the interrelationship of wheat bran, soybean and flax in rats on a high fat diet was evaluated.

A total of 165 male Fischer-344 rats were obtained from Hilltop Laboratories (Scottdale, PA) at 21 days of age. The rats were housed five per cage. After a one week acclimatization and quarantine of consuming standard laboratory rat chow 5001 (Purina), the rats were randomly divided into eleven groups, each group receiving one of the eleven test diets (Table 2) for 10 weeks. Since fat and fibre are important dietary factors in colon carcinogenesis, a basic high fat diet (HFD, contained 20 %w/w of fat) was formulated (Table 2). One percent of wheat bran fibre as part of a HFD was used as a control. This represents an experimental diet in which 26 grams of wheat bran were present in one kilogram of whole diet (wheat bran contains 44% total dietary fibre) . The test diets contained the basic diet together with an equal weight of whole grains to the amount of wheat bran needed for an 8% fibre diet.

**TABLE 1**

| *Basic diet (BD):* | |
|---|---|
| INGREDIENT | grams/kg |
| Casein | 178 |
| DL-Methionine | 2 |
| Dextrose | 184 |
| Sucrose | 184 |
| Fat Mixture* | 200 |
| Salt Mixture** | 35 |
| Vitamin Mixture*** | 10 |
| Choline Bitatrate | 2 |
| total: | 795g/kg |

| | |
|---|---|
| *By weight, the fat is 44.2% lard, 46.2% corn oil, and 9.6% hydrogenated coconut oil. | |
| ** The salt mixture is AIN-76 Salt Mixture. | |
| *** The vitamin mixture is AIN-76A Vitamin Mixture. | |

**TABLE 2**

| *Diets* | | | |
|---|---|---|---|
| COMBINATION | SOY | FLAX | WHEAT BRAN |
| (1) | 0 | 0 | 100% |
| (2) | 100% | 0 | 0 |
| (3) | 0 | 100% | 0 |
| (4) | 1/3 | 1/3 | 1/3 |
| (5) | 1/2 | 0 | 1/2 |
| (6) | 0 | 1/2 | 1/2 |
| (7) | 1/2 | 1/2 | 0 |
| (8) | 1/6 | 1/6 | 2/3 |
| (9) | 2/3 | 1/6 | 1/6 |
| (10) | 1/6 | 2/3 | 1/6 |
| (11) control | 0 | 0 | 1/8 |

Throughout the study, the animals had free access to deionized water ad libitum. Body weights were recorded weekly. At two weeks after the administration of the experimental diet began, the rats were given 2 s.c. injections of AOM (15 mg/kg body weight/week) or saline (vehicle) . After 10 weeks on these diets, all the rats from each group were sacrificed by exsanguination under ether anesthesia, and their colons were removed and prepared for evaluating of colonic aberrant crypt formation.

The test groups were given wheat bran only, or a combination of wheat bran, soybean and flax seed in different ratios as the source of dietary fibre with an equal weight of whole grains to the amount of wheat bran needed for an 8% fibre diet.

The effect is measured as the number of large ACF/colon.

### Results

The following combinations showed an average of 70% greater reduction in inhibiting ACF development than that achieved by wheat bran alone:

| | |
|---|---|
| • 1/2 soy + 1/2 flax | 72.2% |
| • 1/2 soy + 1/2 wheat bran | 69.3% |
| • 2/3 soy + 1/6 flax + 1/6 wheat bran | 68.2% |

The following combinations show an average of 50% greater reduction in ihnibiting ACF development than that achieved by wheat bran alone:

| | |
|---|---|
| . 1/6 soy + 1/6 flax + 2/3 wheat bran | 55.7% |
| . 1/6 soy + 2/3 flax + 176 wheat bran | 55.1% |
| . soy alone | 53.4% |
| . 1/3 soy + 1/3 flax + 173 wheat bran | 43.1% |

### EXAMPLE 2

A further study using the same animal colon cancer model as described above was performed. In this experiment a dietary fibre composition comprising the following ingredients was compared to wheat bran only:

| COMPOSITION | PARTS BY WEIGHT | WT% |
|---|---|---|
| wheat bran | 2 | 28 |
| oat bran | 2 | 28 |
| psyllium | 1 | 14 |
| rice bran | 1 | 14 |
| soy flour | 0.5 | 7 |
| flax seed | 0.5 | 7 |
| almonds | 0.2 | 2 |

As the amount of fibre from the composition according to the invention increases, the relative protective effect is increased.

In the figure it is shown that the above composition according to the invention has a greater inhibitory effect on aberrant crypt foci (ACF) (and colon cancer) than wheat bran alone. The results indicated a doubling of the protective effect when using an amount of the composition of 8% compared to the use of the same amount of wheat bran only.

## Claims

1. Dietary fibre composition comprising any two or three components or derivative subfractions of wheat bran, soy and flaxseed in an amount equivalent to a ratio of from 1:1 to 1:9 parts by weight of any or each component.

2. Dietary fibre composition according to claim 1, further comprising psyllium seed or derivative subfractions thereof in an amount equivalent to a ratio of from 0.1 to 4 parts by weight of the composition.

3. Dietary fibre composition according to claim 1 or 2, further comprising rice bran or derivative subfractions thereof in an amount equivalent to a ratio of from 0.1 to 4 parts by weight of the composition.

4. Dietary fibre composition according to one or more of the preceding claims, further comprising oat bran or derivative subfractions thereof in an amount equivalent to a ratio of from 0.1 to 4 parts by weight of the composition.

5. Dietary fibre composition according to one or more of the preceding claims, further comprising one or more ingredients chosen from the group comprising almonds, vitamins, minerals and phytonutrients.

6. Dietary fibre composition according to one or more of the preceding claims, wherein the composition is cooked.
